# EUROPEAN PATENT APPLICATION

(11) **EP 4 454 497 A1**
(43) Date of publication of application: **30.10.2024**
(21) Application number: 23745985.4
(22) Date of filing: 12.01.2023
(51) Int. Cl.: A24F 40/40, A24F 40/20

(54) **AEROSOL GENERATING DEVICE AND EXTRACTION METHOD**

(30) Priority: 27.01.2022 CN 202210099855
(71) Applicant: Shenzhen First Union Technology Co., Ltd., Shenzhen, Guangdong 518000 (CN)
(72) Inventor: KUAI, Supeng, Shenzhen, Guangdong 518000 (CN); ZHU, Yonghua, Shenzhen, Guangdong 518000 (CN); XU, Zhongli, Shenzhen, Guangdong 518000 (CN); LI, Yonghai, Shenzhen, Guangdong 518000 (CN)
(74) Representative: Jacob, Reuben Ellis
(86) International application number: PCT/CN2023/071946
(87) International publication number: WO 2023/143102

(57) **Abstract**

An aerosol generating device (200) and an extraction method are disclosed, for use in enabling an aerosol generating product (1) to generate an aerosol. The aerosol generating product (1) includes a first segment (11) and a second segment (12). The second segment (12) is exposed outside the aerosol generating device (200) during use. The aerosol generating device (200) includes a main body (2), where the main body (2) includes: a housing (21), defining a receiving cavity (29) for accommodating the first segment (11); a heater (22), configured to heat the aerosol generating product (1) to generate an aerosol; and a protector (23), moving from a first position to a second position along with the first segment (11) in response to an extraction force applied to the second segment (12), where the first position is a stroke start point of the protector (23) inside the aerosol generating device (200), and the second position is a stroke end point of the protector (23) inside the aerosol generating device (200).

## Description

### CROSS-REFERENCE TO RELATED APPLICATIONS

This application claims priority to Chinese Patent Application No. 202210099855.9, filed with the China National Intellectual Property Administration on January 27, 2022 and entitled "AEROSOL GENERATING DEVICE AND EXTRACTION METHOD", which is incorporated herein by reference in its entirety.

### TECHNICAL FIELD

Embodiments of this application relate to the field of aerosol generating technologies, and in particular, to an aerosol generating device and an extraction method.

### BACKGROUND

An existing aerosol generating device usually includes a heating body. In some aerosol generating devices, the heating body can extend into an inhalable product and generate heat inside the inhalable product, thereby volatilizing the inhalable product to generate an aerosol.

However, when generating heat inside the inhalable product, the heating body is easily bonded to the inhalable product due to the high temperature, and the inhalable product baked by the heating body becomes fragile and easily broken. Therefore, when the inhalable product needs to be pulled out, the inhalable product is easily cracked or has residues falling off in a process of being separated from the heating body, making it difficult to clean a receiving cavity for accommodating the inhalable product.

To resolve the problem, an extractor is arranged in some existing aerosol generating devices. During use, the inhalable product is inserted into the extractor. When the inhalable product needs to be separated from the aerosol generating device, the extractor needs to be grabbed, and an extraction force needs to be applied to the extractor, so that the inhalable product is driven to be separated from the heating body by the extractor, and the inhalable product is limited by the extractor, to prevent residues from falling into the receiving cavity.

### SUMMARY

Embodiments of this application provide an aerosol generating device and an extraction method, so that under the protection of a protector, an aerosol generating product can be directly grabbed to be separated from the aerosol generating device.

An embodiment of this application provides an aerosol generating device, for use in enabling an aerosol generating product to generate an aerosol. The aerosol generating product includes a first segment and a second segment, and the second segment is exposed outside the aerosol generating device during use. The aerosol generating device includes a main body, where the main body includes:
a housing, defining a receiving cavity for accommodating the first segment;
a heater, configured to heat the aerosol generating product to generate an aerosol; and
   a protector, moving from a first position to a second position along with the first segment in response to an extraction force applied to the second segment, where the first position is a stroke start point of the protector inside the aerosol generating device, and the second position is a stroke end point of the protector inside the aerosol generating device.

An embodiment of this application provides an aerosol generating device, for use in enabling an aerosol generating product to generate an aerosol. The aerosol generating device includes a main body, where the main body includes:
a housing, defining a receiving cavity for accommodating at least a part of the aerosol generating product;
a heater, at least partially arranged in the receiving cavity, to enter and heat the aerosol generating product; and
a protector, arranged around at least a partial periphery of the aerosol generating product, and moving between a first position and a second position, wherein the first position is a stroke start point of the protector inside the aerosol generating device, and the second position is a stroke end point of the protector inside the aerosol generating device,
wherein when the protector is in the first position and the second position, the heater is at least partially located inside the aerosol generating product.

An embodiment of this application provides a method for extracting an aerosol generating product from an aerosol generating device. The aerosol generating product includes: a first segment including an aerosol-forming substrate and a second segment including a suction nozzle;
the aerosol generating device includes a housing, a heater at least partially arranged inside the housing, and a protector; and
the method includes:
   inserting the aerosol generating product, so that the first segment is connected to the protector in a close fitting manner, and the heater is inserted into the first segment; and
   applying an extraction force to the second segment, so that the protector and the first segment move together in an extraction direction, and after the protector moves to a stroke end point of the protector, the protector stops in the extraction direction, and the first segment keeps moving in the extraction direction, to detach the first segment from the protector.

In the foregoing aerosol generating device and extraction method, the protector can move from the first position to the second position along with the first segment in response to the extraction force applied to the second segment, so that in an extraction process, the aerosol generating product moves relative to the heater under the protection of the protector, and the bonding between the aerosol generating product and the heater can be released or loosened in the movement from the first position to the second position. In this way, under the protection of the protector, the aerosol generating product can be effectively prevented from being cracked or broken in the receiving cavity in a process of being separated from the heater, which facilitates complete extraction of the aerosol generating product from the aerosol generating device. In addition, the aerosol generating product can be extracted by directly grabbing the second segment of the aerosol generating product exposed outside the aerosol generating device, which is more in line with daily usage habits and thinking inertia of users, and can improve user experience.

### BRIEF DESCRIPTION OF THE DRAWINGS

One or more embodiments are exemplarily described with reference to the corresponding figures in the accompanying drawings, and the exemplary descriptions are not to be construed as limiting the embodiments. Elements in the accompanying drawings that have same reference numerals are represented as similar elements, and unless otherwise particularly stated, the figures in the accompanying drawings are not drawn to scale.
FIG. 1 is an overall schematic diagram of an aerosol generating device according to an embodiment of this application;
FIG. 2 is a cross-sectional view of an aerosol generating device equipped with an aerosol generating product according to an embodiment of this application;
FIG. 3 is a cross-sectional view of an aerosol generating device without an aerosol generating product according to an embodiment of this application;
FIG. 4 is a schematic diagram of a main body and a protector in an aerosol generating device after disassembly according to an embodiment of this application;
FIG. 5 is a schematic diagram of a housing of an aerosol generating device according to an embodiment of this application;
FIG. 6 is a schematic partial diagram of an aerosol generating device according to an embodiment of this application; and
FIG. 7 is a partial cross-sectional view of an aerosol generating device according to an embodiment of this application.

In the accompanying drawings:
200: Aerosol generating device;
1: Aerosol generating product; 11: First segment; 111: Aerosol-forming substrate segment; 112: First cooling segment; 12: Second segment; 121: Suction nozzle; 122: Second cooling segment;
2: Main body; 21: Housing; 211: First sliding portion; 22: Heater; 23: Protector; 231: Sleeve; 2311: Groove; 2312: First sleeve; 2313: Second sleeve; 2314: Second sliding portion; 232: Base; 233: Through hole; 234: Close fitting portion; 2341: Barbed structure; 235: Convex rib; 236: Second stop portion; 24: Power supply assembly; 241: Battery cell; 242: Circuit board; 25: Insertion port; 251: Recessed portion 26: First stop portion; 27: Touch screen; 28: Shifting member; 281: Trigger portion; 282: Execution portion;
3: Inductive switch;
41: First magnetic member; 42: Second magnetic member;
5: Movable member

### DETAILED DESCRIPTION

The technical solutions in the embodiments of this application are clearly and completely described below with reference to the accompanying drawings in the embodiments of this application. Apparently, the described embodiments are merely some rather than all of the embodiments of this application. Based on the embodiments of this application, all other embodiments obtained by a person of ordinary skill in the art without creative efforts shall fall within the protection scope of this application.

The terms "first", "second", and "third" in this application are merely intended for a purpose of description, and shall not be understood as indicating or implying relative significance or implicitly indicating the number or order of indicated technical features. All directional indications (for example, upper, lower, left, right, front, and back) in the embodiments of this application are only used for explaining relative position relationships, movement situations, or the like among the various components in a specific posture (as shown in the accompanying drawings). If the specific posture changes, the directional indications change accordingly. In addition, terms "comprise", "have", and any variations thereof are intended to indicate nonexclusive inclusion. For example, a process, method, system, product, or device that comprises a series of steps or units is not limited to the listed steps or units; and instead, further optionally comprises a step or unit that is not listed, or further optionally comprises another step or unit that is intrinsic to the process, method, product, or device.

"Embodiment" mentioned in this specification means that particular features, structures, or characteristics described with reference to the embodiment may be included in at least one embodiment of this application. The term appearing at different positions of the specification may not refer to the same embodiment or an independent or alternative embodiment that is mutually exclusive with another embodiment. A person skilled in the art explicitly or implicitly understands that the embodiments described in this specification may be combined with other embodiments.

It should be noted that, when an element is referred to as "being fixed to" another element, the element may be directly on the another element, or an intervening element may be present. When an element is considered to be "connected to" another element, the element may be directly connected to the another element, or one or more intervening elements may exist between the element and the another element at the same time. The terms "vertical", "horizontal", "left", "right", and similar expressions used in this specification are merely used for purposes of illustration but not indicate a unique implementation.

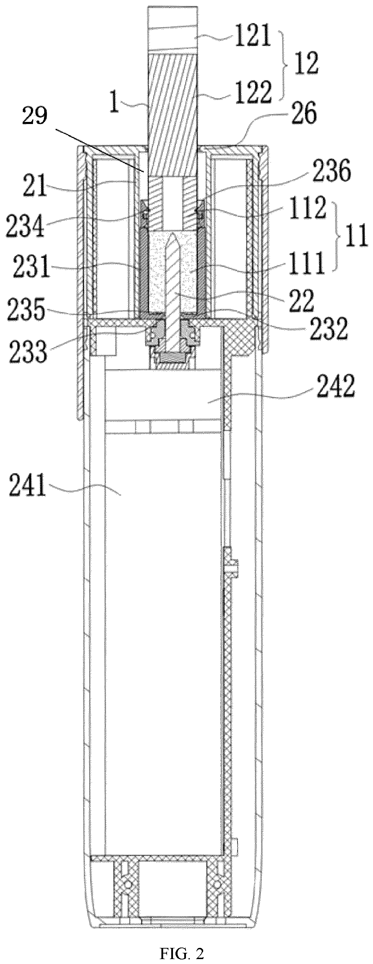

An embodiment of this application provides an aerosol generating device 200. The device may be configured to heat an aerosol generating product, to volatilize the aerosol generating product to generate an aerosol from for inhalation. The aerosol may include Chinese herbal medicines, nicotine, or flavor compounds such as tobacco flavor. In an embodiment shown in FIG. 2, an aerosol generating product 1 is a tobacco product (such as a cigarette or a cigar), but this is not limited.

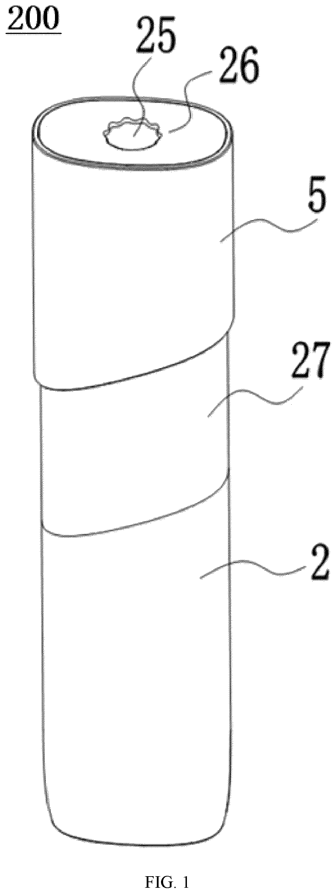

In the embodiments shown in FIG. 1 and FIG. 2, the aerosol generating device 200 includes a receiving cavity 29 for receiving the aerosol generating product 1 and a heater 22 configured to heat the aerosol generating product 1, and further includes a power supply assembly 24. The power supply assembly 24 is configured to supply power to the heater 22.

Referring to FIG. 1 and FIG. 2, a main body 2 includes an insertion port 25. The aerosol generating product 1, for example, the cigarette, is removably received in the receiving cavity 29 through the insertion port 25. At least a part of the heater 22 extends in the receiving cavity 29 in an axial direction, and generates heat by electromagnetic induction under a changing magnetic field, or generates heat through resistance when powered on, or radiates an infrared ray to the aerosol generating product 1 when excited, to further heat the aerosol generating product 1, for example, the cigarette, so that at least one component of the aerosol generating product 1 is volatilized to form an aerosol for inhalation. The power supply assembly 24 includes a battery cell 241 and a circuit board 242. The battery cell 241 is a rechargeable direct current battery cell, and may output a direct current. The circuit board 242 is electrically connected to the rechargeable battery cell 241, and is configured to control output of a current, a voltage, or electrical power of the battery cell 241. In other embodiments, the battery cell 241 may also be a disposable battery, which is not rechargeable or does not need to be charged. In other implementations, the power supply assembly 24 may be a wired power supply. The wired power supply is directly connected to the mains through a plug to supply power to the aerosol generating device 200.

In a preferred embodiment, a direct-current power supply voltage provided by the battery cell 241 ranges from about 2.5 V to about 9.0 V, and an amperage of a direct current that can be provided by the battery cell 241 ranges from about 2.5 A to about 20 A.

Further, in an optional implementation, the aerosol generating product 1 is preferably made of a tobacco-containing material that releases a volatile compound from a substrate when being heated, or a non-tobacco material suitable for electric heating and smoking after being heated. The aerosol generating product 1 is preferably made of a solid substrate. The solid substrate may include one or more of powders, particles, fragmented strips, strips, or flakes of one or more of vanilla leaves, tobacco leaves, homogeneous tobacco, and expanded tobacco. Alternatively, the solid substrate may include additional tobacco or non-tobacco volatile aroma compounds to be released when the substrate is heated. In some optional implementations, the aerosol generating product is prepared to a shape of a conventional cigarette or cigar.

In a preferred implementation, the heater 22 includes magnetically inductive materials that can generate heat in a changing magnetic field such as grade 430 stainless steel (SS430), grade 420 stainless steel (SS420), or an alloy material containing iron and nickel (for example, permalloy), so that the heater can generate heat in the changing magnetic field, then heat itself in the changing magnetic field due to generation of eddy current and magnetic hysteresis, and conduct and/or radiate heat to the aerosol generating product to heat the aerosol generating product. Correspondingly, the aerosol generating device 200 further includes a magnetic field generator, for example, an induction coil, configured to generate a changing magnetic field under an alternating current. The circuit board is connected to the battery cell and the induction coil, and may convert a direct current outputted by the battery cell into an alternating current. Preferably, a frequency of the alternating current ranges from 80 KHz to 400 KHz. More specifically, the frequency may be in a range of approximately 200 KHz to 300 KHz.

In a preferred implementation, the heater 22 has an infrared coating, which can be excited by the direct current provided by the battery cell 241 to radiate an infrared ray, to heat at least a part of the aerosol generating product 1. The infrared coating is preferably formed by oxides of at least one metal element such as Mg, Al, Ti, Zr, Mn, Fe, Co, Ni, Cu, or Cr. These metal oxides radiate a far-infrared ray having a heating effect when power is supplied. A preparation manner may be spraying the above metal oxide on a surface of the substrate by plasma spraying and then solidifying it. When the infrared coating emits the infrared ray to the aerosol generating product 1, the aerosol generating product 1 generates heat, and when a heating temperature reaches a preset temperature, an aerosol may be generated.

In a preferred implementation, the heater 22 is made of a resistive conductive material such as an iron-chromium aluminum alloy, a nickel-chromium alloy, a nickel-iron alloy, platinum, tungsten, silver, or a conductive ceramic, or a conductive material including at least one of the foregoing. In this way, the aerosol generating product 1 may be heated through resistance when conducting electricity, so that at least one component of the aerosol generating product 1 is volatilized to form an aerosol.

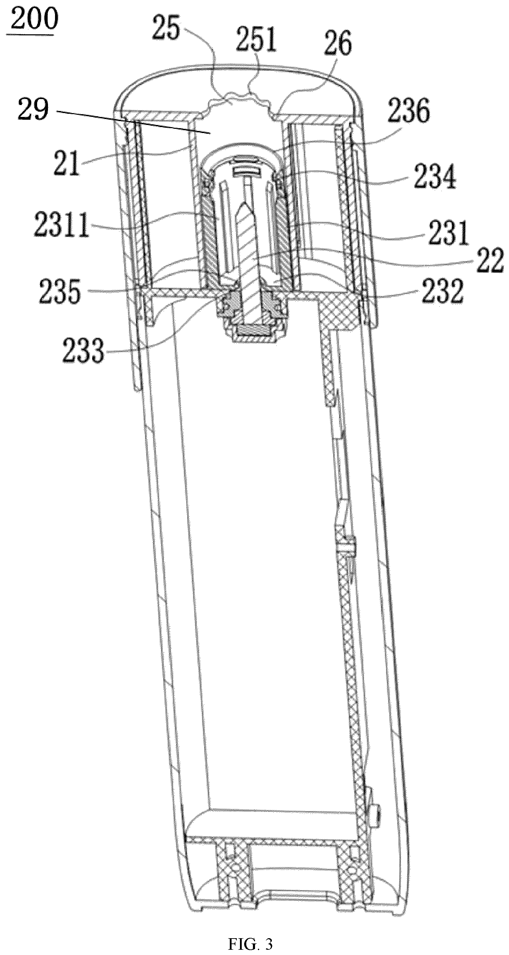

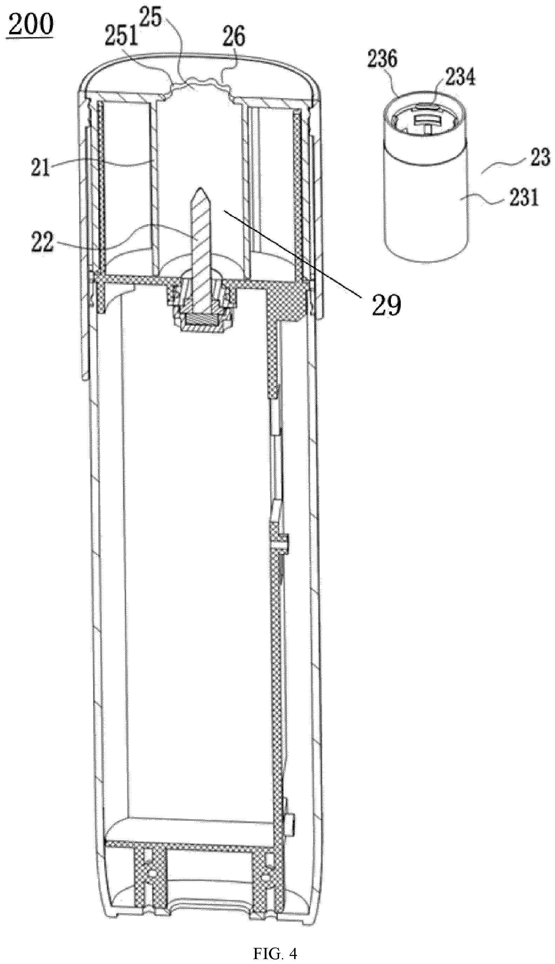

Referring to FIG. 3 and FIG. 4, the heater 22 is generally in a shape of a pin or a needle or a token, which is further beneficial for insertion into the aerosol generating product 1. In addition, the heater 22 may have a length of approximately 12 mm to 19 mm and a diameter of 2.0 mm to 2.6 mm. A cross section of the heater 22 may be a circle, a linear shape, an oval, a polygon, or the like. In other embodiments, the heater 22 may be substantially cylindrical, or another structure that can be arranged at a periphery of the aerosol generating product 1 to conduct and radiate heat from the periphery of the aerosol generating product 1 to the aerosol generating product 1. In this case, at least a part of the protector 23 is located between the heater 22 and the aerosol generating product 1, so that the heater 22 can be avoided from being bonded to the aerosol generating product 1, and the aerosol generating product 1 can be directly extracted by grabbing the exposed second segment 12 of the aerosol generating product 1.

Referring to FIG. 2, the aerosol generating product 1 may be a conventional aerosol generating product, including a first segment 11 and a second segment 12. During use, the first segment 11 can be inserted into the aerosol generating device 200 to the receiving cavity 29, and the second segment 12 remains outside the aerosol generating device 200. The first segment 11 has at least one component such as tobacco, flavoring agents, flavor, and Chinese herb medicines, and is configured to generate an aerosol under the action of the heater 22. The second segment 12 is provided with a suction nozzle 121 for a mouth to hold. The suction nozzle 121 is inhaled through the mouth to enable the aerosol generated by the aerosol generating product 1 to enter the mouth. In some embodiments, the first segment 11 includes an aerosol-forming substrate segment 111 and a first cooling segment 112. The aerosol-forming substrate segment 111 is inserted into the heater 22 and is in contact with the heater 22, and is capable of generating the aerosol under the action of the heater 22. The first cooling segment 112 does not include a substance capable of generating volatiles under the action of the heater 22. A distance between a bottommost end of the first cooling segment 112 and a bottommost end of the aerosol-forming substrate segment 111 is greater than a depth of insertion of the heater 22 into the first segment 11, so that the first cooling segment 112 is located outside a contact range of the heater 22. The first cooling segment 112 has a function of cooling the aerosol, to prevent a temperature of the aerosol entering the mouth from being excessively high to burn the mouth. In some embodiments, the second segment 12 includes a suction nozzle 121 and a second cooling segment 122. The suction nozzle 121 is arranged uppermost, and the second cooling segment 122 mainly has a cooling function, to prevent the temperature of the aerosol entering the mouth from being excessively high to burn the mouth. Certainly, a third segment may be further arranged between the first segment 11 and the second segment 12. The first segment 11 and the second segment 12 are connected by the third segment, so that when an extraction force is applied to the second segment 12, the first segment 11 is movable in an extraction direction in response to the extraction force under the traction of the third segment. The third segment may also have a cooling function. When the suction nozzle 121 or the third segment is sufficiently long, or a cooling effect of the first cooling segment 112 and/or the third segment is sufficiently good, the second cooling segment 122 may be omitted.

In the embodiments shown in FIG. 2 to FIG. 4, the aerosol generating device 200 includes a main body 2. The main body 2 includes a housing 21. An interior of the housing 21 has a cavity. The cavity forms a receiving cavity 29 for accommodating at least a part of the aerosol generating product 1. The main body 2 further includes a protector 23. The protector 23 is used for protecting the aerosol generating product 1, so that the aerosol generating product 1 is not cracked or broken when the aerosol generating product 1 is detached by relative movement from the heater 22, thereby ensuring that the aerosol generating product 1 can be completely pulled out of the aerosol generating device 200. In addition, under the protection of the protector 23, a user may extract the aerosol generating product 1 by directly grabbing the second segment 12 exposed outside the aerosol generating device 200, to detach the aerosol generating product 1 from the aerosol generating device 200.

Referring to FIG. 2 to FIG. 4, the housing 21 is made of a material resistant to high temperature and having a thermal insulation function, such as PEEK, and is arranged around the receiving cavity 29, to prevent the high temperature in the receiving cavity 29 from being radiated from a side to burn a hand, and the housing 21 also helps to keep the high temperature in the receiving cavity 29, thereby reducing energy consumption. In some embodiments, to increase a thermal insulation effect of the housing 21, a wall of the housing 21 has a cavity inside. The cavity may be vacuum or filled with air. The cavity is arranged around the receiving cavity 29 to increase thermal insulation and heat preservation effects.

Referring to FIG. 2 to FIG. 4, the protector 23 can move by a stroke along with the first segment 11 in response to the extraction force applied to the second segment 12. A stroke start point is the first position and a stroke end point is the second position. A stroke trajectory may be linear, helical, bent, curved, or the like.

Specifically, the protector 23 may provide a movable connection between the first segment 11 and the housing 21 defining the receiving cavity 29. That is, the protector 23 is movably connected to the housing 21, so that the protector 23 can move between the first position and the second position on the main body 2. The aerosol generating product 1 is connected to the protector 23, and a connection force between the aerosol generating product 1 and the protector 23 is greater than a connection force between the protector 23 and the housing 21, so that when the extraction force is applied to the aerosol generating product 1, the protector 23 can move from the first position to the second position along with the aerosol generating product 1, and the aerosol generating product 1 moves at least from the first position to the second position in a process in which the protector 23 moves from the first position to the second position. The first position may be a working position. When at the first position, at least a part of the heater 22 is located inside the aerosol generating product 1. The heater 22 heats the aerosol generating product 1, so that when the aerosol generating product 1 generates the aerosol, the aerosol generating product 1 is located in the first position. The first position may be the stroke start point in the movement of the protector 23 and/or the aerosol generating product 1 on the main body 2 in a process of extracting the aerosol generating product 1. The second position is a stroke end point to which the protector 23 moves on the main body 2 from the first position in a process of extracting the aerosol generating product 1. The aerosol generating product 1 and the protector 23 always remain connected between the first position and the second position (including above the first position and the second position). In a process of the protector 23 moving from the first position to the second position, the first segment 11 accommodated in the receiving cavity 29 also moves at least from the first position to the second position, so that the heater 22 gradually exits the first segment 11. Therefore, the bonding between the heater 22 and the first segment 11 is loosened, which facilitates the extraction of the first segment 11 from the heater 22. In addition, the protector 23 protects the first segment 11 by limiting the side wall of the first segment 11 and/or supporting the bottom of the first segment 11, to prevent the first segment 11 from being cracked or broken in a process of exiting the receiving cavity 29 and/or the protector, which facilitates the complete extraction of the aerosol generating product 1 from the aerosol generating device 200. In a process of extracting the aerosol generating product 1, a stroke of the protector 23 is a stroke from the first position to the second position. A stroke of the aerosol generating product 1 includes a stroke from the first position to the second position, and further includes a stroke to release a connection with the protector.

In a preferred embodiment, when the protector 23 is at the first position and the second position, the heater 22 is at least partially located inside the first segment 11. Specifically, when the protector 23 is at the first position and the second position, the heater 22 is at least partially located inside the aerosol-forming substrate segment 111 in the first segment 11. In other words, at the first position and the second position, the first segment 11 remains connected to the heater 22, and the first segment 11 is not completely detached from the heater 22. Reasons for such a design include: 1. The protector 23 protects the first segment 11 between the first position and the second position, so that the first segment 11 can be effectively prevented from being cracked or broken in a process of debonding from the heater 22, which can ensure that the aerosol generating product 1 remains intact in a process of debonding from the heater 22. 2. In a process of the first segment 11 moving from the first position to the second position, it is sufficient to release the bonding formed between the first segment 11 and the heater 22 due to the high temperature. After the bonding is released, the first segment 11 is no longer cracked or broken in a process of being detached from the heater 22, so that the integrity of the first segment 11 can be continuously maintained. Therefore, it is allowed that the first segment 11 at the second position is not detached from the heater 22. 3. By making an axial linear distance between the first position and the second position less than a depth of the first segment 11 when the heater 22 is inserted at the first position, a linear distance between the first position and the second position is limited, which avoids an increase in a volume of the receiving cavity 29 due to the arrangement of the protector 23, and facilitates the miniaturization of the aerosol generating device 200.

In a preferred embodiment, the protector 23 protects a side wall of the first segment 11 by providing a sleeve 231, thereby protecting the first segment 11 and preventing the first segment 11 from being cracked or broken. In the embodiments shown in FIG. 2 and FIG. 3, the protector 23 includes a through hole 233 for the heater 22 to pass through to enter the first segment 11, and further includes a sleeve 231 surrounding the through hole 233 and extending in an axial direction. An interior of the sleeve 231 is for accommodating the first segment 11. The sleeve 231 is in a tubular shape, and a lower end of the sleeve 231 in an axial direction may be completely opened, to form the through hole 233. The sleeve 231 supports and protects the side wall of the first segment 11 in a process of debonding the first segment 11 from the heater 22, to prevent the side wall of the first segment 11 from being cracked or broken. In addition, the sleeve 231 is connected to the side wall of the first segment 11. When an extraction force is applied to the second segment 12, the extraction force is transferred through the second segment 12 and acts on the sleeve 231 through the side wall of the first segment 11, to enable the sleeve 231 to move from the first position to the second position.

An axial length of the sleeve 231 is at least greater than a length of the aerosol-forming substrate segment 111 in the first segment 11. The aerosol-forming substrate segment 111 in the first segment 11 may be completely accommodated inside the sleeve 231. Since the heater 22 mainly bakes the aerosol-forming substrate segment 111, the aerosol-forming substrate segment 111 after baking is more fragile than the first cooling segment 112. To better protect the aerosol-forming substrate segment 111, preferably, at least a part of the first cooling segment 112 is located inside the sleeve 231 and is connected to the sleeve 231 in a close fitting manner. In this way, when the aerosol generating product 1 is extracted upward by grabbing the second segment 12, the side wall of the first cooling segment 112 bears a main acting force of connecting the first segment 11 and the sleeve 231 to each other, and driving the protector 23 to move from the first position to the second position, thereby protecting the side wall of the aerosol-forming substrate segment 111 from being cracked or broken.

Referring to FIG. 2 and FIG. 3, the sleeve 231 is provided with a close fitting portion 234 extending in a radial direction of the sleeve 231 to a center of the sleeve 231. The close fitting portion 234 is connected to the first segment 11 in a close fitting manner, and may form an acting force with the first segment 11 that can grab the side wall of the first segment 11. Specifically, at least a part of the close fitting portion 234 is connected to the first cooling segment 112 in the first segment 11 in a close fitting manner, so that when an upward extraction force is applied to the second segment 12, the protector 23 can grab the first segment 11 by friction force, and can move in the extraction direction along with the first segment 11, that is, to the second position. Under the action of the close fitting portion 234, the protector 23 may remain relatively static and move synchronously with the first segment 11 within a certain stroke. In other words, at least at the first position, the protector 23 and the first segment 11 have the same moving speed. Certainly, it is not excluded that, under the action of the extraction force on the second segment 12, while both the protector 23 and the first segment 11 move in a direction of the second position, the first segment 11 may also move in a direction of the second position along the side wall of the sleeve 23. In other words, under the action of the extraction force, a moving speed of the first segment 11 is greater than a moving speed of the protector 23. When the moving speed of the first segment 11 in the extraction direction is greater than the moving speed of the sleeve 231, there is relative movement between the first segment 11 and the sleeve 231 to separate from each other. For example, when the protector 23 is at the second position, the moving speed in the extraction direction changes to 0, and the moving speed of the first segment 11 in the extraction direction is greater than 0, so that the first segment 11 may perform a separation movement from the sleeve 231. In an optional embodiment, after the moving speed of the protector 23 in the extraction direction at the second position decreases to 0, the speed may be maintained at 0 until after the first segment 11 is completely detached from the protector 23, the protector 23 automatically moves to the first position in the extraction direction, that is, automatically resets after being completely separated from the first segment. In another optional embodiment, after the moving speed of the protector 23 in the extraction direction at the second position decreases to 0, the protector 23 may immediately move in a reverse direction to the first position under the action of a repulsive force or another force.

In some embodiments, since the close fitting portion 234 has a large friction force coefficient, a large static friction force may be formed between the close fitting portion 234 and the side wall of the first segment 11 when the close fitting portion 234 is in contact with the side wall of the first segment 11. Therefore, there is no need to make the close fitting portion 234 be a protrusion extending to the center of the sleeve 231.

In a preferred embodiment, the close fitting portion 234 is made of a flexible material such as silica gel. When the first segment 11 is inserted into the sleeve 231, the close fitting portion 234 is in an interference fit with the first segment 11, to implement a close fitting connection and generate a close fitting force such as a friction force or an elastic force. The close fitting force applied by the close fitting portion 234 to the first segment 11 is greater than an acting force between the sleeve 231 and the housing 21 and the gravity of the protector 23, so that the protector 23 can be pulled to move from the first position to the second position. In addition, the close fitting force is less than a maximum bearing force of the aerosol generating product 1 in an axial direction, so that the aerosol generating product 1 is not pulled apart when the aerosol generating product 1 is extracted.

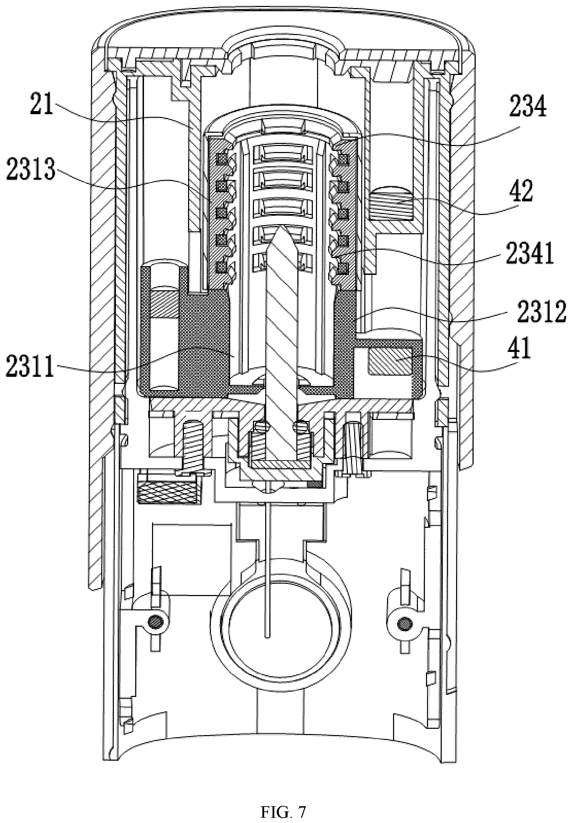

Referring to FIG. 7, the close fitting portion 234 includes a barbed structure 2341 extending in a radial direction of the sleeve 231 and obliquely downward. The barbed structure 2341 is located on a lower end surface of the close fitting portion 234. An upper end surface of the close fitting portion 234 may extend obliquely downward, that is, is arranged to conform to a direction in which the first segment 11 is inserted into the sleeve 231, to facilitate insertion of the first segment 11 into the sleeve 231. The barbed structure 2341 is inclined downward inside the sleeve 231 or is located inside the sleeve 231 and extends in a radial direction, and faces a direction opposite to a direction in which the first segment 11 exits the first sleeve 231, thereby helping to hinder the first segment 11 from exiting the sleeve 231, and increasing the acting force between the sleeve 231 and the first segment 11.

Referring to FIG. 7, the sleeve 231 includes a first sleeve 2312 and a second sleeve 2313. The second sleeve 2313 is made of a flexible material such as silica gel, and has a larger friction force coefficient than that of the first sleeve 2312. The second sleeve 2313 may be integrally formed with the close fitting portion 234, or may replace the close fitting portion 234 to be directly connected to the first segment 11, and generate an acting force that hinders the first segment 11 from exiting the sleeve 231. The first sleeve 2312 is made of a hard material such as PEEK, and partially extends to a region in which the second sleeve 2313 is located, to connect and support the second sleeve 2313. The second sleeve 2313 extends in an axial direction, and may be connected to a side wall of the first cooling segment 112 and/or a side wall of a part of the aerosol-forming substrate segment 111. For example, a part of the close fitting portion 234 may be connected to the side wall of the first cooling segment 112, and the remaining part of the close fitting portion 234 may be connected to the side wall of the aerosol-forming substrate segment 111.

In a preferred embodiment, the protector 23 protects and supports a bottom of the first segment 11 by providing a base 232. The base 232 supports the first segment 11 upward, thereby preventing the first segment 11 from being cracked or residues from falling off in a process of debonding the first segment 11 from the heater 22. In the embodiments shown in FIG. 2 and FIG. 3, the base 232 extends in a radial direction from a bottom of the sleeve 231. The through hole 233 for the heater 22 to pass through runs through the base 232 and may be located at a center of the base 232.

An aperture of the through hole 233 may be slightly greater than an outer diameter of the heater 1, so that when the protector 23 moves to the second position, that is, the heater 22 moves back from the through hole 233 relative to the protector 23, an edge of the through hole 233 can scratch residues of the aerosol generating product 1 attached to a surface of the heater 22, to clean the heater 22.

To prevent the aerosol from entering a region other than the interior of the protector in the receiving cavity 29 through air convection to contaminate the region, in the embodiment shown in FIG. 3, the main body further includes an insertion port 25. The first segment 11 passes through the insertion port 25 and is then inserted into the receiving cavity 29. At least one recessed portion 251 is provided on an edge of the insertion port 25. The recessed portion 251 is for the air to pass through, so that the air may enter the receiving cavity 29 through the recessed portion 251. Referring to FIG. 3, a plurality of recessed portions 251 is uniformly or symmetrically distributed on the edge of the insertion port 25, so that the air can enter the receiving cavity 29 from a plurality of directions or positions.

Referring to FIG. 3, a first air passage in communication with the insertion port 25 is provided on the sleeve 231. A second air passage in communication with the first air passage is provided on the base 232. The recessed portion 251 on the insertion port 25, the first air passage, and the second air passage provide an air path for air to enter the aerosol generating product 1. The air may enter the aerosol generating product 1 from the side wall of the aerosol generating product 1 and/or enter the aerosol generating product 1 from the bottom of the aerosol generating product 1.

In a further embodiment, referring to FIG. 3, the base 232 has a ring of convex ribs 235 arranged around a periphery of the through hole 233. The convex ribs 235 extend in the axial direction, and may further support the bottom of the aerosol generating product 1. The second air passage is jointly defined by the base 232 and the convex ribs 235. Referring to FIG. 3, a groove 2311 extending in an axial direction is provided on an inner wall of the sleeve 231. An upper end of the groove 2311 is in communication with the recessed portion 251 on the insertion port 25. A lower end of the groove 2311 is in communication with the base 232, to be in communication with the second air passage. The first air passage is defined by the groove 2311. The groove 2311 may have a plurality of strips, and may be symmetrically or uniformly distributed on an inner side wall of the sleeve 231. Specifically, referring to FIG. 7, a large number of mesh holes are provided on the first sleeve 2312, for the close fitting portion 234 to pass through and extend into the first sleeve 2312. The second sleeve 2313 is arranged at a partial periphery of the first sleeve 2312. The groove 2311 is provided on an inner side wall of the first sleeve 2312.

During inhalation through the suction nozzle 121, the air enters the receiving cavity 29 through the recessed portion 251 on the insertion port 25, enters the second air passage through the first air passage, then enters a first end of the aerosol generating product 1 from the bottom of the aerosol generating product 1 through the second air passage, and finally enters the mouth through the suction nozzle 121 on the second segment 12. Certainly, when there is an air hole on the side wall of the aerosol generating product 1 or when the side wall of the aerosol generating product 1 is permeable to air, the air in the first air passage may enter the aerosol generating product 1 through the side wall of the aerosol generating product 1.

In the embodiments shown in FIG. 2 and FIG. 3, the sleeve 231 has a complete side wall. Therefore, the groove 2311 needs to be formed on the side wall of the sleeve 231 to form at least a part of the first air passage. In some other embodiments, the side wall of the sleeve 231 is a hollowed-out side wall, which may be provided with a hollowed-out hole, or the side wall of the sleeve 231 includes one or more claw-shaped or sheet-shaped or post-shaped structures extending upward from the base 232. The structure is arranged at a periphery of the first segment 11 and connected to at least a part of the side wall of the first segment 11. The air may enter the sleeve 231 through the hollowed-out hole, or through a gap between the claw-shaped or sheet-shaped or post-shaped structures, and then enter the first segment 11.

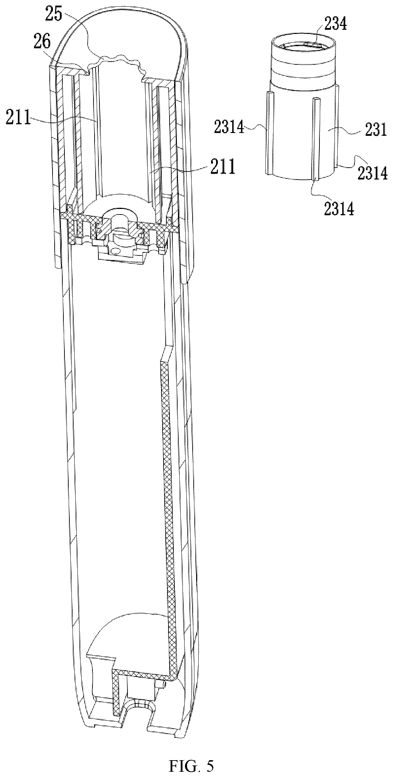

The protector 23 can move from the first position to the second position by sliding relative to the housing 21. Referring to FIG. 4 and FIG. 5, the housing 21 is slidably connected to the sleeve 231.

In a preferred embodiment, as shown in FIG. 4 and FIG. 5, a shape of an inner wall of the housing 21 is appropriate for a shape and a size of an outer wall of the protector 23. The outer wall of the protector 23 fits the inner wall of the housing 21, and when the protector 23 moves to the second position or the first position, the protector 23 slides along the inner wall of the housing 21. In an embodiment, as shown in FIG. 4, the outer wall of the protector 23 is smoothly in contact with the inner wall of the housing 21, to reduce a sliding friction force between the outer wall of the protector 23 and the inner wall of the housing 21, and ensure that a friction force between the outer wall of the protector 23 and the inner wall of the housing 21 is less than a close fitting force between the aerosol generating product 1 and the close fitting portion 234, so that when the aerosol generating product 1 is extracted by grabbing the second segment 12, the protector 23 can move along with the aerosol generating product 1, to move to the second position together. To further reduce movement resistance when the outer wall of the protector 23 and the inner wall of the housing 21 move relative to each other, a ball may be arranged between the outer wall of the protector 23 and the inner wall of the housing 21, so that the outer wall of the protector 23 is in rolling contact with the inner wall of the housing 21. In another embodiment, to further reduce the movement resistance when the outer wall of the protector 23 and the inner wall of the housing 21 move relative to each other, a contact area between the outer wall of the protector 23 and the inner wall of the housing 21 may be reduced. For example, a convex strip is provided on the outer wall of the protector 23, and the convex strip is smoothly connected to the inner wall of the housing 21, or a convex strip is provided on the inner wall of the housing 21, and the outer wall of the protector 23 is smoothly connected to the convex strip, thereby reducing the friction force.

In another preferred embodiment, as shown in FIG. 5, the housing 21 is provided with a first sliding portion 211. The sleeve 231 is provided with a second sliding portion 2314 slidably connected to the first sliding portion 211. One of the first sliding portion 211 and the second sliding portion 2314 is a guide rail guiding a sliding direction, and the other is a sliding block or a sliding groove sliding along the guide rail. The guide rail limits the sliding direction, which can prevent the protector from moving off the guide rail. The guide rail may be linear or helical. To simplify a movement trajectory of the protector 23 in the receiving cavity 29, preferably, the guide rail extends parallel to an axial direction of the receiving cavity 29, so that the guide rail is linear, to prevent the protector 23 from rotating in the receiving cavity 29, thereby reducing a torque between the protector 23 and the side wall of the aerosol generating product 1, thereby protecting the aerosol generating product 1 from being cracked or broken. Certainly, in other embodiments, another mechanism is further arranged on the main body 2 or the protector 23, for preventing the protector 23 from rotating when the protector 23 moves from the first position to the second position. Certainly, it is not excluded that in some embodiments, there is no contact between the protector 23 and the housing 21. For example, the protector 23 may be at least partially suspended in the receiving cavity 29 in the housing 21.

In some embodiments, when the protector 23 is at the first position or the second position, or at a position between the first position and the second position, the protector 23 may at least partially extend out of the receiving cavity 29, thereby facilitating cleaning and maintenance for the protector 23.

However, to prevent the user from mistouching the protector 23 and to prevent the user from being burned by the protector 23, the protector 23 may be hidden inside the aerosol generating device 200 without being exposed. In the embodiment shown in FIG. 3, the protector 23 does not extend out of the receiving cavity 29 when at the first position and the second position, and does not extend out of the receiving cavity 29 when at another position between the first position and the second position, so that the user cannot touch the protector 23 without disassembly, thereby protecting the user, and improving the safety factor of the aerosol generating device 200.

A stop portion may be arranged to limit the protector 23 in the receiving cavity 29, to prevent the protector 23 from being exposed to the aerosol generating device 200 beyond the receiving cavity 29. In the embodiment shown in FIG. 3, a first stop portion 26 is arranged on the main body 2, and a second stop portion 236 is arranged on the protector. The first stop portion 26 is used for preventing the second stop portion 236 from keeping moving in the extraction direction, thereby stopping the protector 23 at the second position.

In an optional embodiment, when the protector 23 is at the second position, the first stop portion 26 is in contact with the second stop portion 236, the first stop portion 26 is located downstream of the second stop portion 236 in the extraction direction, and the first stop portion 26 prevents the protector 23 having the second stop portion 236 from keeping moving by contacting the stop position. After the protector 23 reaches the second position, the protector 23 may stay at the second position to wait for a new aerosol generating product 1 to be inserted into the protector 23, and with the insertion of the new aerosol generating product 1, the protector 23 moves to the first position under the pushing of the new aerosol generating product 1, and finally stays at the first position. Then the heater 22 may heat the aerosol generating product 1. Both the first stop portion 26 and the second stop portion 236 are magnetic members, or a magnetic material and a magnetic member to each other. The magnetic member described herein includes a permanent magnet or an electromagnet that can generate a magnetic field. The magnetic material described herein includes a material that can be attracted by the magnetic member, such as iron, so that when the first stop portion 26 and the second stop portion 236 close to each other, the first stop portion 26 and the second stop portion 236 have a suction force therebetween. When a distance between the first stop portion 26 and the second stop portion 236 is less than a threshold, the suction force is greater than the gravity of the protector 23, so that after the protector 23 is pulled out of the aerosol generating product 1, the protector 23 can be maintained at the second position. When the protector 23 inserted with the aerosol generating product 1 is at the first position, the suction force between the first stop portion 26 and the second stop portion 236 is less than the gravity of the protector 23 or less than a sum of the gravity of the protector 23 and the gravity of the aerosol generating product 1, so that the protector 23 and the aerosol generating product 1 can be maintained at the first position. When an extraction force is further applied to the second segment 12 under the action of the suction force, the protector 23 and the aerosol generating product 1 can more easily and effortlessly move from the first position to the second position, so that a smaller extraction force can be used, which reduces difficulty of extraction, and helps to reduce the acting force between the protector 23 and the side wall of the aerosol generating product 1 when moving from the first position to the second position, and can further prevent the aerosol generating product 1 from being cracked or broken in a process of moving from the first position to the second position. In a more further embodiment, when at the first position, a weight of the aerosol generating product 1 gradually decreases due to the generation of the aerosol. When the weight of the aerosol generating product 1 decreases to a threshold (for example, when the generated aerosol no longer meets a requirement for each inhalation, or the aerosol can no longer be generated), the suction force between the first stop portion 26 and the second stop portion 236 is greater than a sum of the gravity of the protector 23 and the gravity of the aerosol generating product 1 in this state, so that the protector 23 may drive the aerosol generating product 1 to automatically move from the first position to the second position, to provide a better user experience.

After the aerosol generating product 1 is pulled out of the protector 23, when the protector 23 can be maintained at the second position, an inductive switch may be arranged in the main body 2. When the inductive switch 3 induces the returning of the protector 23 to the first position, the inductive switch 3 is triggered, so that an electrical connection between the circuit board 242 and the heater 22 is conducted to enable the heater 22 to generate heat or the like. A manner of conducting the heater includes: 1. After the inductive switch 3 is triggered, a signal is transmitted to a circuit switch between the heater 22 and the power supply assembly 24, to directly control the circuit switch to be turned on, thereby conducting an electrical connection between the heater 22 and the power supply assembly 24; or 2. after the inductive switch 3 is triggered, a signal is transmitted to a processor inside the aerosol generating device 200, and the processor then outputs a new signal to control a circuit switch between the heater 22 and the power supply assembly 24 to be turned on, thereby conducting an electrical connection between the heater 22 and the power supply assembly 24; or when the inductive switch 3 is triggered, the aerosol generating device 200 is automatically started, so that the aerosol generating device 200 is in a standby state (even if the heater 22 is in a pre-started state). When the aerosol generating device 200 is in the standby state, the aerosol generating device 200 may be controlled through a button or a touch screen 27 arranged on the aerosol generating device 200, for example, the heater 22 is controlled to generate heat, or when the aerosol generating device 200 is in the standby state, the aerosol generating device 200 may be controlled through a remote control to execute a corresponding command or the like, for example, the heater 22 is controlled to generate heat. By setting the protector 23 in the first position as a condition for triggering the inductive switch 3, an inappropriate group such as children can be prevented from incorrectly starting the aerosol generating device 200.

The inductive switch 3 may be a piezoelectric switch, and is arranged at a bottom of the receiving cavity 29. When the protector 23 is at the first position, the inductive switch 3 is pressed or compressed by the protector 23 to be triggered. The inductive switch 3 may include a magnetic induction sensor or a magnetic switch (such as a Hall switch or a magnetic flux sensor), and is triggered by a magnetic field strength exceeding a specific threshold. In an optional solution, the inductive switch 3 is arranged on the main body 2 corresponding to the first position. A first magnetic member 41 (such as a magnet) for generating a magnetic field is arranged on the protector 23. When the protector 23 is closer to the first position, the inductive switch 3 is subjected to a stronger magnetic field generated by the first magnetic member 41. When the strength reaches an induction threshold of the inductive switch 3, the inductive switch 3 is triggered. In a process of extracting the aerosol generating product 1, the protector 23 moves gradually away from the inductive switch 3 from the first position to the second position, so that the magnetic field strength sensed by the inductive switch 3 gradually decreases. When the strength is less than the induction threshold of the inductive switch 3, the inductive switch 3 is turned off. In another optional solution, the inductive switch 3 is arranged on the protector 23. Since the inductive switch 3 is small and light, the weight of the protector 23 is not greatly increased. The first magnetic member 41 (for example, a magnet) is arranged on the main body 2 corresponding to the first position. When the protector 23 is closer to the first position, the inductive switch 3 is subjected to a stronger magnetic field generated by the first magnetic member 41. When the strength reaches the induction threshold of the inductive switch 3, the inductive switch 3 is triggered. Otherwise, the inductive switch 3 is turned off. In an optional solution, the inductive switch 3 is arranged on the main body 2 corresponding to the first position. A trigger is arranged in the aerosol generating product. When the first segment 11 is at the first position, the trigger is induced by the inductive switch 3, so that the inductive switch 3 is triggered. Certainly, it is not excluded that, there is another inductive switch 3 that can determine whether the protector 23 reaches the first position and can be triggered when the protector 23 reaches the first position.

In another optional embodiment, when the protector 23 is at the second position, the first stop portion 26 and the second stop portion 236 are in no contact or no direct contact with each other. For example, both the first stop portion 26 and the second stop portion 126 are magnetic members, and when close to each other, the first stop portion 26 and the second stop portion 126 have a repulsive force to each other. The repulsive force is greater than an interaction force between the protector 23 and the aerosol generating product 1, so that when the second stop portion 236 cannot or hardly contact the first stop portion 26, it can be ensured that the aerosol generating article 1 can be pulled out of the protector 23. After the aerosol generating product 1 is completely detached from the protector 23 under the action of the repulsive force and the gravity of the protector 23, the protector 23 may automatically reset, that is, automatically return to the first position from the second position.

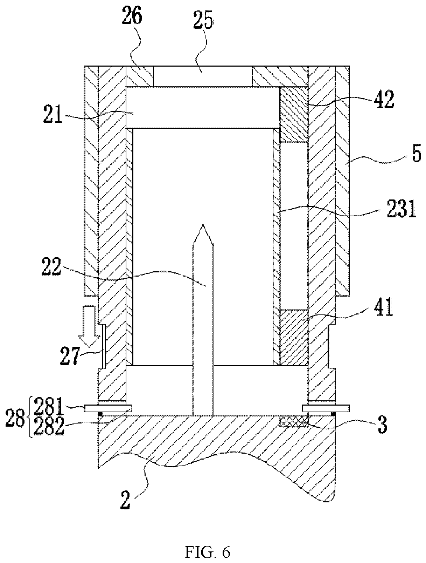

In a more preferred solution, referring to FIG. 6, the main body 2 includes a first magnetic member 41 and a second magnetic member 42. The first magnetic member 41 (for example, a magnet) and the second magnetic member 42 (for example, a magnet) can provide a magnetic field, and the first magnetic member 41 and the second magnetic member 42 have a magnetic force when closing to each other. The inductive switch 3 is arranged on the main body 2 corresponding to the first position, and the first magnetic member 41 is arranged on the protector 23. The second magnetic member 42 is arranged on the main body 2 corresponding to the second position. When the protector 23 is at the first position, a magnetic field strength applied by the first magnetic member 41 to the inductive switch 3 triggers the inductive switch 3, and when the protector 23 is at the second position, a magnetic attraction force between the first magnetic member 41 on the protector 23 and the second magnetic member 42 on the main body 2 maintains the protector 23 at the second position (therefore, in some embodiments, a magnetic induction material that can be attracted by the first magnetic member 41 may be used to replace the second magnetic member 42). Certainly, the second magnetic member 42 may be arranged on the first stop portion 26 or near the first stop portion 26.

In another optional embodiment, an elastic member, such as a spring, is arranged between the first stop portion 26 and the second stop portion 236, or at least one of the first stop portion 26 and the second stop portion 236 is an elastic member. When the protector 23 is at the second position, the first stop portion 26 and the second stop portion 236 are elastically connected. The elastic member has a buffering and anti-collision function, which can prevent the second stop portion 236 from hitting the first stop portion 26 when the extraction force is excessive, causing the movement to stop, and then causing cracking or breaking of the aerosol generating product 1.

In the embodiment shown in FIG. 3, the main body 2 includes the insertion port 25 for the first segment 11 to enter the receiving cavity 29. The first stop portion 26 is arranged axially above the receiving cavity 29 and extends in the radial direction. The insertion port 25 runs through the first stop portion 26, that is, the insertion port 25 is located on the first stop portion 26. A minimum distance from a center to an edge of the insertion port 25 is less than a minimum distance between a center of the receiving cavity 29 and the housing 21. In this way, at least a part of the first stop portion 26 is suspended above the receiving cavity 29 to stop the second stop portion 236.

In a case that a surface of the heater 22 is not cleaned in time for long-term use, there may be a larger bonding force between the aerosol generating product 1 and the heater 22 after heating, so that it is difficult to extract the aerosol generating product 1 partially inserted into the aerosol generating device 200 through the second segment 12 of the aerosol generating product 1, and if the aerosol generating product 1 is extracted forcibly, the aerosol generating product 1 may be cracked. To resolve the problem, in an embodiment, referring to FIG. 6, the main body 2 further includes a shifting member 28. The shifting member 28 includes a trigger portion 281 and an execution portion 282. The execution portion 282 is arranged corresponding to the protector 23, to push the protector 23 in a direction of the second position when the trigger portion 281 is triggered, to loosen the connection between the heater 22 and the aerosol generating product 1. The trigger portion 281 may be a control mechanism, such as a button on the aerosol generating device 200 or an operation instruction on the touch screen 27, for generating a control signal. The execution portion 282 belongs to an electric mechanism and is electrically connected to the circuit board 242. When the circuit board 242 obtains a corresponding control signal, the execution portion 282 is started. The execution portion 282 then performs a response action based on the control signal to push the protector 23. The trigger portion 281 and the execution portion 282 may belong to a mechanical linkage mechanism, and are connected to each other through a link, a lever, a gear, or the like. In this way, when the trigger portion 281 is triggered, a movement and/or an acting force is transferred through the link, the lever, the gear, or the like, so that the execution portion 282 pushes the protector 23 upward. Specifically, the aerosol generating device 200 further includes a movable member 5 movably connected to the main body 2. The movable member 5 may include a sliding cover and be arranged at a periphery of the main body 2, to be able to slide in an axial direction of the main body 2, or slide in a lateral direction or a circumferential direction of the main body 2. The trigger portion 281 is arranged on a movement trajectory of the movable member 5. When the movable member 5 slides to a third position along the movement trajectory of the movable member 5, the movable member 5 may trigger the trigger portion 281 by abutting, pressing, or shielding light.

To prevent the user from mistouching the button or the touch screen 27 arranged on the aerosol generating device 200 when the movable member 5 moves relative to the main body 2, in the embodiment shown in FIG. 6, the touch screen 27 and/or the button may be arranged on the movement trajectory of the movable member 5. When the movable member 5 slides to a fourth position along the movement trajectory of the movable member 5, the switch and/or the touch screen 27 may be blocked at least partially by the movable member 5. Preferably, after the movable member 5 reaches the fourth position, and when the movable member 5 keeps moving along the movement trajectory in a movement direction of the movable member 5, the movable member 5 reaches the third position, to trigger the trigger portion 281. It is not excluded that, in other embodiments, after the trigger portion 281 is triggered, the heater 11 moves back under the action of the motor, thereby loosening the bonding between the heater 11 and the first segment 11.

Certainly, in other embodiments, the shifting member 28 may not be arranged. However, to prevent the user from mistakenly triggering the aerosol generating device 200 or starting the aerosol generating device 200 when the button or the touch screen 27 arranged on the aerosol generating device 200 is temporarily not needed, a movable member may be arranged on the main body 2, and the movable member 5 may slide on the main body 2 to the fourth position, to block a mechanism, such as the button or the touch screen 27, that may trigger or start the aerosol generating device 200.

In a more specific embodiment, the movable member 5 includes a cylindrical structure, which is at least partially sleeved around a periphery of the housing 21, and slidably connected to the periphery of the housing 21, and can slide up and down relative to the housing 21 in an axial direction. The third position and/or the fourth position are arranged in an axial direction of the main body 2. When the cylindrical structure is at the third position or the fourth position, an outer side wall of the housing 21 is at least partially exposed. In a regular state, the outer side wall of the housing 21 is hidden by being shielded by the cylindrical structure. Optionally, the cylindrical structure triggers the movement trajectory of the trigger portion 281 or blocks a trajectory of the button or the touch screen 27 in a direction opposite to a movement trajectory of the protector 23 moving from the first position to the second position. Preferably, when the cylindrical structure is at the third position, the cylindrical structure still remains blocking the button or the touch screen 27.

An embodiment of this application further provides a method for extracting an aerosol generating product from the aerosol generating device 200. The method includes:
(1) The aerosol generating product 1 is inserted, so that the first segment 11 of the aerosol generating product 1 is connected to the protector 23 in a close fitting manner. Preferably, the sleeve 231 of the protector 23 is arranged at a periphery of the aerosol-forming substrate segment 111 in the first segment 11 to protect and support the side wall of the aerosol-forming substrate segment 111. The close fitting portion 234 on the sleeve 231 is connected to the first cooling segment 112 on the first segment 11 that is not baked at the high temperature by the heater 22 in a close fitting manner. An interaction force between the sleeve 231 and the first segment 11 is mainly borne by the first cooling segment 112. The heater 22 needs to be inserted into the first segment 11 to heat the aerosol-forming substrate segment 111.
(2) The second segment 12 of the aerosol generating product 1 exposed outside the aerosol generating device 200 is grabbed. A manner of grabbing the second segment 12 is simple, and the second segment 12 may be directly grabbed by fingers. Then, an extraction force for extracting the aerosol generating product 1 from the aerosol generating device 200 is applied to the second segment 12, to enable the protector 23 and the first segment 11 to move from the first position to the second position in the extraction direction. In a process of moving from the first position to the second position, the protector 23 and the aerosol generating product 1 may maintain simultaneous movement at the same speed.
(3) When the protector 23 moves to the stroke end point and stops keeping moving in the extraction direction, the extraction force is continuously applied to the second segment 12, to detach the first segment 11 from the protector 23, until the first segment 11 is pulled out of the protector 23, thereby completing the extraction of the aerosol generating product 1 from the aerosol generating device 200.

In the aerosol generating device and the extraction method described above, the protector provides a movement connection between the first segment and the housing forming the receiving cavity, so that in an extraction process, the aerosol generating product may move relative to the housing through the protector. In this way, when the bonding between the aerosol generating product and the heater is loosened, the protector can support and protect the aerosol generating product, to prevent the aerosol generating product from being cracked or broken in the receiving cavity, which facilitates complete extraction of the aerosol generating product from the aerosol generating device. In addition, the aerosol generating product can be extracted by directly grabbing the second segment of the aerosol generating product exposed outside the aerosol generating device, which is more in line with daily usage habits and thinking inertia of users, and can improve user experience.

An extractor is arranged in an existing aerosol generating device. During use, the aerosol generating product is first grabbed, and then the aerosol generating product is assembled into the extractor. When the aerosol generating product needs to be pulled out of the aerosol generating device, the extractor needs to be started (including electrically starting the extractor or manually directly grabbing the extractor), to enable the extractor to move relative to the receiving cavity, and then the aerosol generating product is grabbed, to separate the aerosol generating product from the extractor. In other words, when the aerosol generating product is extracted, at least two actions of starting the extractor and grabbing the aerosol generating product are required. According to the solutions provided in this application, the aerosol generating product may be directly inserted into the aerosol generating device by holding the second segment of the aerosol generating product. When the aerosol generating product needs to be pulled out of the aerosol generating device, the aerosol generating product may be pulled out of the aerosol generating device by directly holding the second segment of the aerosol generating product. In comparison with the existing aerosol generating device, this application is simpler to use. Moreover, according to the thinking inertia of users, when the aerosol generating product needs to be pulled out, it is easy to avoid the extractor to directly grab the aerosol generating product, and directly apply the extraction force to the aerosol generating product. As a result, the extractor arranged in the existing aerosol generating device is not effectively utilized, and it is difficult to avoid causing the aerosol generating product to be cracked in the extractor or causing a part of the aerosol generating product to remain in the extractor, reducing user experience. The solutions provided in this application comply with the thinking inertia of users, and can prevent the aerosol generating product from being cracked (being cracked includes that tobacco in the cigarette is detached from a tobacco paper surrounding the tobacco in the cigarette). In the protector, relatively complete extraction of the aerosol generating product can be ensured, to provide a better user experience.

It should be noted that, the specification of this application and the accompanying drawings thereof illustrate preferred embodiments of this application, but this application is not limited to the embodiments described in the specification. Further, a person of ordinary skill in the art may make improvements or variations according to the foregoing description, and all the improvements and variations shall fall within the protection scope of the appended claims of this application.

## Claims

1. An aerosol generating device, for use in enabling an aerosol generating product to generate an aerosol, wherein:
the aerosol generating product comprises a first segment and a second segment;
the second segment is exposed outside the aerosol generating device during use;
the aerosol generating device comprises a main body; and
the main body comprises:
a housing defining a receiving cavity for accommodating the first segment;
a heater configured to heat the aerosol generating product to generate an aerosol; and
a protector moving from a first position to a second position along with the first segment in response to an extraction force applied to the second segment, wherein:
the first position is a stroke start point of the protector inside the aerosol generating device; and
the second position is a stroke end point of the protector inside the aerosol generating device.

2. The aerosol generating device according to claim 1, wherein the heater is in contact with the first segment when at either the first position or the second position.

3. The aerosol generating device according to claim 1, wherein the protector does not extend out of the receiving cavity when at either the first position or the second position.

4. The aerosol generating device according to claim 1, wherein:
the protector comprises a sleeve and a base;
the sleeve is arranged around the base; and
the base is provided with a through hole for the heater to pass through to enter the first segment.

5. The aerosol generating device according to claim 4, wherein:
the main body further comprises an insertion port for the first segment to enter the receiving cavity;
a first air passage in communication with the insertion port is arranged on the sleeve;
a second air passage in communication with the first air passage is arranged on the base; and
the insertion port, the first air passage, and the second air passage provide an air path for air to enter the aerosol generating product.

6. The aerosol generating device according to claim 1, wherein:
the protector comprises a close fitting portion for fitting the first segment and generating an acting force; and
the protector moves to the second position together with the first segment by the acting force.

7. The aerosol generating device according to claim 6, wherein the close fitting portion is at least partially made of a flexible material.

8. The aerosol generating device according to claim 6, wherein the close fitting portion is a protrusion extending to a center of the sleeve in a radial direction.

9. The aerosol generating device according to claim 6, wherein a lower end surface of the close fitting portion is located inside the sleeve to form a barbed structure.

10. The aerosol generating device according to claim 6, wherein:
the first segment comprises an aerosol-forming substrate segment for generating an aerosol and a first cooling segment for cooling the aerosol;
the first cooling segment is located beyond a contact range of the heater; and
at least a part of the close fitting portion is for contacting the first cooling segment.

11. The aerosol generating device according to claim 4, wherein:
the housing is slidably connected to the sleeve; or
at least a part of the sleeve is suspended inside the housing.

12. The aerosol generating device according to claim 11, wherein:
a first sliding portion is arranged on the housing;
a second sliding portion slidably connected to the first sliding portion is arranged on the sleeve; and
one of the first sliding portion and the second sliding portion is a guide rail for guiding a sliding direction.

13. The aerosol generating device according to claim 12, wherein the guide rail extends parallel to an axial direction of the receiving cavity.

14. The aerosol generating device according to claim 1, wherein:
a first stop portion is arranged on the main body;
a second stop portion is arranged on the protector, and
the first stop portion is used for stopping the second stop portion from keeping moving in an extraction direction, so that the protector stops at the second position.

15. The aerosol generating device according to claim 13, wherein:
the main body further comprises an insertion port for the first segment to enter the receiving cavity;
the first stop portion is arranged at a top end of the receiving cavity in an axial direction and extends in a radial direction; and
the insertion port runs through the first stop portion.

16. The aerosol generating device according to claim 1, wherein:
the main body further comprises a second magnetic member for generating a magnetic field;
the second magnetic member is arranged near the second position;
the protector is at least partially made of a magnetic induction material or is provided with a first magnetic member; and
when the protector is at the second position, a magnetic attraction force between the first magnetic member and the second magnetic member remains the protector at the second position.

17. The aerosol generating device according to claim 16, wherein:
the main body further comprises a magnetic induction sensor or a magnetic switch;
the magnetic induction sensor or the magnetic switch is arranged near the first position;
the protector is provided with the first magnetic member; and
when the protector is located at the first position, the magnetic induction sensor transmits a signal to a processor in response to a magnetic field change brought by the first magnetic member to start or pre-start the heater, or the magnetic switch conducts an electrical connection between a power supply and the heater to start the heater.

18. An aerosol generating device, for use in enabling an aerosol generating product to generate an aerosol, wherein the aerosol generating device comprises a main body, and the main body comprises:
a housing defining a receiving cavity for accommodating at least a part of the aerosol generating product;
a heater, at least partially arranged in the receiving cavity, to enter and heat the aerosol generating product; and
a protector, arranged around at least a partial periphery of the aerosol generating product, and moving between a first position and a second position, wherein:
the first position is a stroke start point of the protector inside the aerosol generating device;
the second position is a stroke end point of the protector inside the aerosol generating device;
when the protector is in either the first position or the second position, the heater is at least partially located inside the aerosol generating product.

19. A method for extracting an aerosol generating product from an aerosol generating device, wherein:
the aerosol generating product comprises: a first segment comprising an aerosol-forming substrate and a second segment comprising a suction nozzle;
the aerosol generating device comprises a housing, a heater at least partially arranged inside the housing, and a protector; and
the method comprises:
inserting the aerosol generating product, so that the first segment is connected to the protector in a close fitting manner, and the heater is inserted into the first segment; and
applying an extraction force to the second segment, so that the protector and the first segment move together in an extraction direction, and after the protector moves to a stroke end point of the protector, the protector stops in the extraction direction, and the first segment keeps moving in the extraction direction, to detach the first segment from the protector.

20. The method according to claim 18, wherein the inserting the aerosol generating product comprises:
the aerosol generating product pushing the protector to move from an opposite direction of the stroke end point to a stroke start point of the protector.

21. The method according to claim 18, wherein after the second segment is detached from the protector, the protector automatically moves from an opposite direction of the stroke end point back to a stroke start point.

22. The method according to claim 18, wherein when the protector and the first segment move together in the extraction direction, a moving speed of the first segment in the extraction direction is greater than or equal to a moving speed of the protector in the extraction direction.
